# EUROPÄISCHE PATENTSCHRIFT

(11) **EP 1 726 296 B1**
(45) Veröffentlichungstag und Bekanntmachung des Hinweises auf die Patenterteilung: **03.03.2010**
(21) Anmeldenummer: 06114160.2
(22) Anmeldetag: 18.05.2006
(51) Int. Cl.: A61K 8/44, A61K 8/68, A61K 8/97, A61K 31/195, A61Q 19/08, A61Q 19/00

(54) **Kosmetisches oder therapeutisches Kombinationspräparat mit Theanin**
Cosmetic or therapeutic combination preparation comprising theanine
Préparation combinée cosmétique ou thérapeutique contenant de la theanine

(30) Priorität: 25.05.2005 DE 102005024040
(43) Veröffentlichungstag der Anmeldung: 29.11.2006
(73) Patentinhaber: ROVI Cosmetics International GmbH, 36381 Schlüchtern (DE)
(72) Erfinder: Blume, Gabriele, 36396 Steinau a. d. Strasse (DE); Teichmüller, Dirk, 63589, Linsengericht (DE)
(74) Vertreter: Weber, Roland

(56) Entgegenhaltungen:
- WO-A-03/072118
- WO-A-2004/039348
- WO-A-2005/046623
- DE-A1- 19 929 485
- US-A1- 2003 083 380
- PATENT ABSTRACTS OF JAPAN Bd. 2003, Nr. 12, 5. Dezember 2003 (2003-12-05) & JP 2004 043428 A (KOSE CORP), 12. Februar 2004 (2004-02-12)
- PATENT ABSTRACTS OF JAPAN Bd. 1995, Nr. 07, 31. August 1995 (1995-08-31) & JP 07 107969 A (NIPPON SUISAN KAISHA LTD), 25. April 1995 (1995-04-25)

## Beschreibung

Die vorliegende Erfindung betrifft ein kosmetisches oder therapeutisches Kombinationspräparat mit einem Anteil an im wesentlichen freien Aminosäuren. Darüber hinaus betrifft diese Erfindung die Verwendung eines solchen Kombinationspräparats zur Herstellung eines Kosmetikums für die Verbesserung der Hautfeuchtigkeit, der Hautelastizität und/oder für die Hautfaltenreduzierung.

Die menschliche Haut bildet eine Barriere zwischen der Umgebung und dem Körperinneren. Gleichzeitig ist diese Barriere selbstverständlich auch Kontaktfläche, d.h. daß die Haut durch den Kontakt mit der Umgebung auch ständig Beanspruchungen ausgesetzt ist. Diese Beanspruchungen können sowohl physikalisch (Strahlung, mechanische Belastung) als auch chemisch (Kontakt mit Flüssigkeiten, wie z.B. Seifenlauge) oder biologisch (Bakterien, Pilze, Insektenstiche) sein. Diese umweltbedingten Beanspruchungen führen in der Regel zu einer Beschleunigung des natürlichen Alterungsprozesses der menschlichen Haut.

Auf der Suche nach geeigneten Mitteln, die die Alterungsprozesse der Haut verlangsamen, abstoppen und/oder bestenfalls sogar rückgängig machen können, ist das primäre Ziel in der Regel, ein Mittel zu finden, das insbesondere die Außenwirkung des Alterungsprozesses, v.a. die Faltenbildung, vermindert, vermeidet oder rückgängig macht. Um dies zu erreichen wurden bisher bereits verschiedene Ansätze verfolgt.

Das bekannteste und älteste Prinzip von Antifaltenmitteln ist das Zuführen von geeigneten Fetten. Sogenannte rückfettende Präparate sollen der Faltenbildung vorbeugen, indem der Haut die Fette zugeführt werden, die ihr durch die alltägliche Beanspruchung, z.B. durch häufiges Händewaschen, entzogen worden sind. Ein weiteres Prinzip ist das der sogenannten Feuchtigkeitscremes. Diese sollen vornehmlich den Feuchtigkeitshaushalt der Haut positiv beeinflussen, da angenommen wird, daß die Hautalterung insbesondere mit einer unzureichenden Feuchtigkeit der Haut korreliert.

Die vorgenannten Produkte sind zweifelsfrei in gewissem Umfang wirksam, liefern jedoch das gewünschte Ergebnis nicht nachhaltig genug. Vielmehr ist es bei diesen Produkten meist erforderlich daß diese wenigstens einmal täglich angewandt werden. Häufig wird außerdem beanstandet, daß nach der Beendigung der Anwendung dieser Produkte sehr schnell wieder der Zustand wie vor der Behandlung eintritt bzw. teilweise sogar ein schlechterer Zustand als vorher. D.h., daß in vielen Fällen von einer Verlangsamung der Alterungsprozesse der Haut und vor allem von einer Rückbildung der bereits ausgebildeten Hautfalten nicht wirklich die Rede sein kann, sondern vielmehr von einer lediglich vorübergehenden Faltenglättung. Bei Feuchtigkeitscremes und rückfettenden Präparaten kann somit ein zufriedenstellender Zustand üblicherweise nur erzielt werden, wenn die Produkte täglich, teilweise sogar mehrfach täglich angewandt werden.

Weitere Antifaltenmittel, die auf dem Markt sind, wirken, indem sie die Hornhautoberfläche beschleunigt abtragen, teilweise sogar eine direkte Hautablösung bewirken (Peeling) und gleichzeitig die Neubildung der behandelten Haut anregen. Zu diesem Zweck werden in entsprechende kosmetische Präparate häufig Fruchtsäuren eingearbeitet. Diese Fruchtsäuren sind jedoch nicht bei jedem Hauttyp ohne weiteres anwendbar und es sind viele Fälle bekannt, in denen die Anwendung dieser Mittel zu Hautreizungen führte.

JP 2004 043428 A beschreibt ein wasserhaltiges Pulverkosmetikum, welches sich beim Einreiben verflüssigt und angenehm auf die Haut auflegt und hierbei ein weiches Hautgefühl verleiht. Das Pulverkosmetikum setzt sich zusammen aus einem hydrophobisierten Kieselsäureanhydrid, einem Alkohol mit mehreren Hydroxylgruppen, Wasser und einem Anteil von 0,1 bis 10 Gew.-%, bestehend aus einer oder mehreren Substanzen, die ausgewählt sind unter Histidin, Serin, Glycin, Glycylglycin, Trimethylglycin, Glycylglycylglycin, Theanin, Asparaginsäure, Arginin, Pyroglutaminsäure, Glutaminsäure, Alanin, Valin, Leucin, Isoleucin, Threonin, Tyrosin, Tryptophan, Cystin, Cystein oder Lysin oder Salzen davon.

US 2003/083380 offenbart eine Zusammensetzung, die aus einer Kombination von einem amphoteren oder pseudo-amphoteren Mittel auf der einen Seite und einer Alpha-Hydroxysäure, einer Alpha-Ketosäure oder einer verwandten Verbindung auf der anderen Seite besteht, wobei die Zusammensetzung in einem pharmazeutisch verträglichen Träger für die topische Behandlung von kosmetischen Zuständen oder dermatologischen Störungen vorliegt. Das in dieser Zusammensetzung vorliegende amphotere oder pseudo-amphotere Mittel kann bei bestimmten Ausführungsformen eine Aminosäure sein, die eine beliebige unter einer Vielzahl von Aminosäuren sein kann.

In WO 2004/039348 A wird eine Formulierung für die topische Applikation beschrieben, die ein wasserfreies Trägermedium, eine hohe Konzentration an mikronisierter bioaktiver Substanz und ein Peeling-Mittel enthält, wobei die bioaktive Substanz eine beliebige unter einer Vielzahl von Aminosäuren sein kann.

Die herkömmlichen Antifaltenmittel liefern entweder nicht die gewünschte Faltenglättung bei gleichzeitiger Verlangsamung der Alterungsprozesse der Haut und Rückbildung der bereits ausgebildeten Hautfalten oder führen zu unangenehmen, teilweise gar schmerzlichen Hautreizungen.

Es besteht daher ein Bedarf nach einem Antifaltenmittel, das die Nachteile der vorgenannten herkömmlichen Präparate überwindet, also insbesondere eine nachhaltige Verbesserung der Hautfeuchtigkeit sowie eine nachhaltige Steigerung der Hautelastizität sowie der Hautfestigkeit bewirkt, ohne daß hierfür eine allzu häufige, wie z.B. tägliche, Applikation notwendig ist, sondern gegebenenfalls die Anwendung auch über längere Zeiträume (einige Tage) ausbleiben kann, ohne daß sich der Hautzustand gleich wieder deutlich verschlechtert.

Diese Aufgabe wird erfindungsgemäß gelöst durch ein kosmetisches oder therapeutisches Kombinationspräparat mit einem Anteil an im wesentlichen freien Aminosäuren, wobei der Anteil an im wesentlichen freien Aminosäuren wenigstens Theanin, Asparaginsäure, Glutaminsäure, Glycin, Threonin, Alanin und Serin umfaßt, wobei der Anteil an Theanin bezogen auf den Gesamtanteil der im wesentlichen freien Aminosäuren im Präparat 80 bis 95 Gew.-% beträgt und wobei der Anteil an Asparaginsäure, Glutaminsäure, Glycin, Threonin, Alanin und Serin bezogen auf den Gesamtanteil der im wesentlichen freien Aminosäuren im Präparat insgesamt 5 bis 20 Gew.% beträgt..

Für das Kombinationspräparat der erfindungsgemäßen Zusammensetzung konnte nachgewiesen werden, daß es die Hautfeuchtigkeit und die Elastizität der Haut in dem behandelten Bereich nach zweiwöchiger Behandlung bereits signifikant verbessert. Darüber hinaus konnte nach zwei Wochen Behandlung in dem behandelten Bereich eine deutlich erkennbare Verringerung der Anzahl feiner Fältchen der Haut festgestellt werden. Außerdem war auch eine deutliche Glättung der entsprechenden Hautpartien erkennbar. Desweiteren konnte festgestellt werden, daß die positiven Effekte, die durch Anwendung des Präparats gemäß der Erfindung ersielt werden, mehrere Tage anhalten und auch eine Woche nach Beendigung der Behandlung noch feststellbar sind. Weitere Details zu diesen positiven Eigenschaften des erfindungsgemäßen Kombinationspräparats gehen aus den weiter unten aufgeführten Beispielen hervor.

Ein Großteil der Hautfalten entsteht allein aufgrund von Reflexbewegungen von Hautpartien einschließlich der darunter liegenden Gewebeschichten. Auf diese Weise entstehen z.B. Falten auf der Stim und Lachfalten. Spannungszustände in der Haut, die zur Ausbildung auch von kleineren Falten führen können, werden aber beispielsweise auch durch starken (v.a. kalten) Wind, Luftzug oder auch durch Niederschläge, wie z.B. Regen, ausgelöst. Um einer nervösen Verspannung von auf diese Weise beanspruchten Hautpartien entgegenzuwirken, ist dem erfindungsgemäßen Kombinationspräparat die Aminosäure Theanin zugesetzt. Theanin ist eine Aminosäure, die für die Teepflanze *Camellia sinensis* charakteristisch ist, in deren Blättern in hohen Anteilen als freie Aminosäure enthalten ist. Dieser Aminosäure wird zugeschrieben, daß sie physische Verspannungen lösen kann. So haben Untersuchungen z.B. gezeigt, daß Theanin die krampfauslösende Wirkung des Koffeins hemmen kann.

Allerdings können die positiven Effekte des Theanins vermutlich nur die Faltenbildung verringern und nicht den gesamten Symptomenkomplex der Hautalterung ausgleichen. Daher wurde nach weitern Zusätzen für ein neues Mittel zur vorbeugenden und therapeutischen Behandlung der Alterungsprozesse der menschlichen Haut gesucht. Für diese Suche wurde angenommen, daß es in der belebten Natur einige Organismen geben sollte, deren äußere Abschlußgewebe aufgrund ihrer Lebensweise besonders stark beansprucht werden. Ein Lebensraum, in dem solche Organismen vermutet wurden, schien unter anderem die Gezeitenzone der Meere zu sein, da hier durch Ebbe und Flut zum einen regelmäßig eine hohe mechanische Beanspruchung der Abschlußschichten der dort lebenden Organismen auftritt und zudem ein ständiger Wechsel zwischen Trocken- und Feuchtperioden stattfindet. Darüber hinaus sollte der Organismus auch in der Lage sein, starke Temperaturschwankungen in seiner Umgebung zu ertragen.

Eine Region, in die alle vorgenannten Anforderungen erfüllte, ist die Gezeitenzone der Atlantikküste Namibias. Dies ist u.a. der Lebensraum der auf Steinen und Felsen sessil vorkommenden Grünalge *Enteromorpha intestinalis.* Diese Alge ist zum einen an das Leben in stark salzhaltigem Wasser angepaßt, zum anderen aber auch sehr widerstandsfähig gegenüber langen Trockenperioden (während des Zeitraums der Ebbe) und gegenüber starken Temperaturschwankungen (Temperaturen zwischen 2 und 30°C).

Es wird angenommen, daß die Widerstandsfähigkeit dieser Alge durch bestimmte Biomoleküle (z.B. Proteine) begründet ist, die die hohen Beanspruchungen ausgleichen können. Um dies herauszufinden, wurden für diese Alge charakteristische Proteine aus der Alge extrahiert und enzymatisch hydrolysiert. Der hierdurch erhaltene aufbereitete Extrakt (nachfolgend als Algenhydrolysat bezeichnet) ist reich an natürlichen Aminosäuren und enthält keine Salze. Insbesondere werden in dem Algenhydrolysat folgende freie Aminosäuren mit den in Klammern angegebenen Anteilen, bezogen auf den Gesamtaminosäuregehalt des Hydrolysats (=100%) gefunden: Asparaginsäure (13-18 Gew.-%), Glutaminsäure (2-17 Gew.-%), Alanin (8-12 Gew.-%) und ein Anteil von jeweils 6-9 Gew.-% an Threonin, Glycin und/oder Serin.

Es wurde nun herausgefunden, daß diese Aminosäurenkombination als Grundrezept für ein neuartiges Antifaltenmittel geeignet ist. Beispielsweise ist der spezielle von *Enteromorpha intestinalis* etablierte Aminosäurencocktail geeignet, zum einen den zellulären Stoffwechsel anzukurbeln und zum anderen den Zustand von Zell- und Gewebestützstrukturen, wie z.B. die fibrillenbildenden Gerüsteiweiße Kollagen, Elastin und Keratin, die auch als Skleroproteine bezeichnet werden, zu verbessem.

Auffällig ist beispielsweise, daß das vorgenannte Algenhydrolysat sehr hohe Anteile an Asparaginsäure und Glutaminsäure enthält. Von diesen beiden Aminosäuren ist bekannt, daß sie maßgeblich in den Stoffwechsel der Zellen eingreifen. So ist Asparaginsäure an der Umsetzung von Kohlenhydraten in Energie beteiligt, so daß ein Asparaginsäuremangel zu einer Abnahme der in einer Zelle vorhandenen Energie führen kann. Glutaminsäure ist ebenfalls am Kohlenhydratstoffwechsel beteiligt und wirkt darüber hinaus auch am Fettstoffwechsel mit sowie an den Syntheseprozessen für DNA, Glutathion und andere Aminosäuren. Darüber hinaus sind Glutaminsäure und Asparaginsäure wesentliche Bestandteile des Kollagen. Es wird daher vorgeschlagen, daß wenigstens eine dieser beiden Aminosäuren im erfindungsgemäßen Präparat enthalten sein muß.

Darüber hinaus weist das Algenhydrolysat auch einen beachtlichen Anteil an weiteren Kollagenbausteinen auf. So sind die Aminosäuren Alanin und Glycin in hohen Anteilen vertreten, also die Aminosäuren, die allein 36% eines Kollagenpolymers ausmachen. Gleichsam sind Glycin und Alanin auch die wesentlichen Bausteine des Skleroproteins Elastin, in welchem sie etwa 50% der polymeren Struktur ausmachen. Auch hinsichtlich des Skleroproteins Keratin stellt das vorgenannte Algenhydrolysat einen Großteil der wesentlichen Bausteine bereit. Zu diesen Aminosäuren zählen das im Algenhydrolysat enthaltene Serin, die Glutaminsäure und Asparaginsäure. Darüber hinaus ist im Algenhydrolysat auch die Aminosäure Threonin mit beachtlichen Anteilen enthalten. Bei dieser Aminosäure handelt es sich um eine essentielle Aminosäure, die vom menschlichen Körper nicht synthetisiert werden kann, so daß sie regelmäßig über die Nahrung aufgenommen werden muß. Auch diese Aminosäure ist eine wesentliche Komponente von Skleroproteinen und insbesondere auch von Bedeutung als Bestandteil der Zellmembran.

Es wird angenommen, daß die Zuführung von Skleroproteinbausteinen (möglicherweise im Überschuß) zu einer Verschiebung des Gleichgewichts Synthese/Abbau von Skleroproteinen hin zur Seite der Synthese bewirkt. Es ist jedoch auch möglich, daß durch das Bereitstellen dieser Aminosäuren lediglich ein systemimmanent chronischer Mangel an diesen Bausteinen ausgeglichen wird. In jedem Fall ist es erforderlich, daß das erfindungsgemäße Kombinationspräparat wenigstens zwei der Gerüstbausteine Alanin, Glycin, Threonin und Serin enthält. Welche der vier zur Auswahl stehenden Aminosäuren verwendet werden hängt in erster Linie davon ab, welches Skleroprotein verstärkt gebildet werden soll und inwieweit auch die Zellmembranen gestärkt werden sollen. Die Kombination von Alanin und Glycin bietet sich beispielsweise an, wenn vornehmlich die Kollagen- und/oder Elastinbildung bzw. -erhaltung gefördert werden soll. Der Zusatz von Serin zielt auf die Keratinförderung und Threonin wird allgemein zur Skleroproteinförderung und zur Zellmembranoptimierung eingesetzt.

Bei dem erfindungsgemäßen Kombinationspräparat umfaßt der Anteil an im wesentlichen freien Aminosäuren die Aminosäuren Asparaginsäure, Glutaminsäure, Glycin, Threonin, Alanin und Serin. Besonders bevorzugt werden diese Aminosäuren in Form eines Algenhydrolysats verwendet, das vorzugsweise aus dem Extrakt einer Grünalge hergestellt wurde. Das heißt, daß bei einer bevorzugten Ausführungsform der vorliegenden Erfindung die Aminosäuren in Form eines solchen Algenhydrolysats mit der diesem Algenhydrolysat eigenen Aminosäurezusammensetzung dem Präparat zugesetzt werden. Besonders bevorzugt wird hierfür ein Hydrolysat einer Grünalge der Gattung *Enteromorpha* verwendet. Insbesondere wird das Hydrolysat der Grünalge *Enteromorpha intestinalis* bevorzugt.

Vorzugsweise beträgt der Anteil der im wesentlichen freien Aminosäuren bezogen auf das fertige Präparat 0,5 bis 10 Gew.%. Bei speziellen Ausführungsformen beträgt dieser Anteil 3 bis 6 Gew.-%, und besonders bevorzugt sind Anteile an im wesentlichen freien Aminosäuren am fertigen Kombinationspräparat von etwa 5 Gew.-%. Bezogen auf diesen Gesamtanteil der im wesentlichen freien Aminosäuren im Präparat beträgt der Anteil an Theanin 80-95 Gew.%. Besonders bevorzugt sind Theanin-Anteile im Bereich von 85-95 Gew.-%, und insbesondere werden Theanin-Anteile von 90 bis 94 Gew.-% bevorzugt. Der Anteil des Kombinationspräparats, den die im wesentlichen freien Aminosäuren Asparaginsäure, Glutaminsäure, Glycin, Threonin, Alanin und Serin ausmachen, beträgt bezogen auf den Gesamtanteil der im wesentlichen freien Aminosäuren im Präparat 5 bis 20 Gew.-%. Bevorzugt beträgt dieser Anteil 5 bis 15 Gew.-% und besonders bevorzugt beträgt die Summe der Anteile der vorgenannten Aminosäuren, bezogen auf den Gesamtanteil der im wesentlichen freien Aminosäuren im Präparat, 6 bis 10 Gew.-%.

Bezogen auf den Gesamtanteil der im wesentlichen freien Aminosäuren im Präparat, die von Theanin verschieden sind, beträgt bei besonders bevorzugten Ausführungsformen der Asparaginsäuregehalt 13 bis 18 Gew.-%. Bei weiteren Ausführungsformen beträgt der Glutaminsäuregehalt 12 bis 17 Gew.-% und bei alternativen Ausführungsformen der Alaningehalt 8 bis 12 Gew.- %. Besonders bevorzugt ist es, wenn alle der drei vorgenannten Aminosäuren in den angegebenen Anteilsbereichen im Kombinationspräparat enthalten sind. Weiterhin ist es bevorzugt, wenn bezogen auf den Gesamtanteil der im wesentlichen freien Aminosäuren im Präparat die Anteile von Threonin, Glycin und/oder Serin jeweils 6 bis 9 Gew.-% betragen.

Da manche Grünalgenhydrolysate neben den vorgenannten Aminosäuren auch beachtliche Anteile an Prolin, Leucin und/oder Phenylalanin aufweisen, enthalten spezielle bevorzugte Ausführungsformen der Erfindung bezogen auf den Gesamtanteil der im wesentlichen freien Aminosäuren im Präparat, die von Theanin verschieden sind, jeweils auch 5 bis 8 Gew.-% Prolin, Leucin und/oder Phenylalanin. Insbesondere sind Prolin- und/oder Leucinanteile bevorzugt, da vermutet wird, daß auch diese Aminosäuren als bedeutende Bausteine von Skleroproteinen für eine weitere Verbesserung der Wirkung des erfindungsgemäßen Präparats verantwortlich sein können. Der Prolinanteil im Kollagen beträgt etwa 20%, im Elastin etwa 12 %. Leucin ist mit etwa 4 % im Kollagen und mit 12 % im Elastin enthalten. Auch im Keratin tragen Prolin und Leucin in größerem Umfang zum Aufbau des Moleküls bei.

Unter im wesentlichen freien Aminosäuren werden hier solche Aminosäuren verstanden, die nicht fester Bestandteil eines Peptids sind und die vorzugsweise ungebunden vorliegen. Unter im wesentlichen freien Aminosäuren werden jedoch auch solche Aminosäuren verstanden, die über Wasserstoffbrückenbindungen bzw. ionische Bindungen oder Van-der-Waals-Kräfte mit anderen Substanzen verbunden sind, sowie solche Aminosäuren, die reversibel kovalent mit Trägerstoffen verbunden sind und von den Trägerstoffen spätestens am Wirkort, z.B. durch hydrolytische Spaltung der Bindung freigesetzt werden. Bei speziellen Ausführungsformen sind die im wesentlichen freien Aminosäuren aber gerade wenigstens teilweise in dem Präparat an einem Trägerstoff oder an verschiedene Trägerstoffe gebunden. Entscheidend ist dabei nur, daß am Wirkort, also in der Haut bzw. in den Zellen, die Aminosäuren letztlich ohne weiteres in ihrer tatsächlich freien Form vorliegen.

Da die in dem erfindungsgemäßen Kombinationspräparat notwendigerweise und/oder vorzugsweise enthaltenen Aminosäuren relativ bis stark polar sind, ist es bevorzugt, daß das Kombinationspräparat zusätzlich einen kosmetisch verträglichen Träger umfaßt, der den Transport durch die oberen Hautschichten und durch die Zellmembranen in die Zellen optimiert, damit die Bioverfügbarkeit der wirksamen Substanzen so hoch wie möglich ist. Vorzugsweise umfaßt ein solcher Träger membranbildende Lipide. Besonders bevorzugt ist es, wenn diese membranbildenden Lipide vesikulär vorliegen. Hierfür eignen sich besonders die membranbildenden Lipide, die aus den Gruppen der Phospholipide, Ceramide und Diazylglycoside ausgewählt sind. Weiterhin sind Kombinationspräparate mit membranbildenden Lipiden bevorzugt, die Gemische von Substanzen aus den vorgenannten Gruppen umfassen.

Bei besonders bevorzugten Ausführungsformen der vorliegenden Erfindung enthalten die membranbildenden Lipide wenigstens 70 Gew.-% Phosphatidylcholin und besonders bevorzugt etwa 80 bis 90 Gew.-% Phosphatidylcholin. Eine weitere bevorzugte Ausführungsform ist dadurch gekennzeichnet, daß der Träger zusätzlich Linolsäure in stabilisierter Form enthält. Vorzugsweise enthält das Kombinationspräparat die stabilisierte Linolsäure in einer Menge von 3,5 bis 6,5 Gew.-%.

Darüber hinaus umfaßt das Kombinationspräparat vorzugsweise wenigstens einen kosmetisch verträglichen Hilfsstoff. Als Hilfsstoffe kommen die üblicherweise für kosmetische oder therapeutische Präparate verwendeten Hilfsstoffe in Betracht. Bei bevorzugten Ausführungsformen sind als Hilfsstoff Methylparaben, Sorbinsäure und/oder Kaliumdihydrogenphosphat im Kombinationspräparat enthalten.

Wie bereits erwähnt, weisen die erfindungsgemäßen Kombinationspräparate hervorragende Wirkungen auf die Alterungsprozesse der menschlichen Haut auf. Daher werden die in einem erfindungsgemäßen Kombinationspräparat enthaltenen wirksamen im wesentlichen freien Aminosäuren, nämlich Theanin und eine Aminosäure, die unter Asparaginsäure und Glutaminsäure ausgewählt ist, und zusätzlich wenigstens zwei Aminosäuren, die unter Alanin, Glycin, Threonin und Serin ausgewählt sind, zur Herstellung eines Kosmetikums für die Verbesserung der Hautfeuchtigkeit, der Hautelastizität und/oder für die Hautfaltenreduzierung verwendet.

Besonders bevorzugt ist es, die erfindungsgemäße Aminosäurenkombination wenigstens teilweise, d.h. wenigstens Theanin ausgenommen, aus Algenmaterial, beispielsweise aus einem Proteinextrakt der Grünalge *Enteromorpha intestinalis* herzustellen.

Die Gewinnung der Proteine aus der Alge erfolgt nach Verfahren, die dem Fachmann bekannt sind. Zum Aufschliessen der aus der Alge gewonnen Proteine wird vorzugsweise keine saure Hydrolyse angewandt, da hierbei beim anschließenden Neutralisieren des entstandenen Hydrolysates große Mengen an Salz entstehen. So entstehen bei der sauren Hydrolyse mit Salzsäure beispielsweise große Mengen an Kochsalz, die im Präparat unerwünscht sind und nur schwer aus dem Aminosäurengemisch zu entfernen sind. Daher werden die Algenproteine bei einem bevorzugten Verfahren zur Herstellung eines erfindungsgemäßen Kombinationspräparats enzymatisch mit Proteasen in deren einzelne Aminosäuren zerlegt. Dieses Verfahren verhindert außerdem chemische Reaktionen und Umwandlungen der Aminosäuren, die bei einem niedrigen pH-Wert, der sich durch Zugabe von Säure bei der sauren Hydrolyse einstellt, entstehen können.

Ein besonders bevorzugtes Kombinationspräparat gemäß der vorliegenden Erfindung ist daher insbesondere dadurch gekennzeichnet, daß das in dem Präparat enthaltene Algenhydrolysat oder das in dem Präparat enthaltene Hydrolysat des Algenextrakts im wesentlichen frei von Salzen ist.

Bei einem besonders bevorzugten Verfahren zur Herstellung eines erfindungsgemäßen Kombinationspräparats werden zunächst die im wesentlichen freien Aminosäuren in geeigneten Mengen unter Rühren in Wasser bei maximal 40°C klar gelöst. Anschließend wird diese Lösung mit einer ethanolischen Lecithinlösung vereinigt und die beiden Lösungen werden langsam unter Turraxieren (= Homogenisieren unter Verwendung eines Turrax-Homogenisators) zusammengeführt und anschließend durch Hochdruckhomogenisation, Extrusion und/oder anderweitige mechanische Zerkleinerung auf eine Vesikeldurchmessergröße von etwa 100 bis 250 nm gebracht. Unter stetigem Homogenisieren wird dann wäßriger Phosphatpuffer zugesetzt und solange weiter homogenisiert, bis eine leicht viskose, homogene Emulsion entsteht. Der pH-Wert der Emulsion wird erforderlichenfalls mit herkömmlichen Mitteln auf etwa pH 5,5 bis 7,0 eingestellt. Auf diese Weise wird in der Regel ein gelblich-braunes Fluid erhalten, das einen leichten Ethanolgeruch und einen charakteristischen Lecithingeruch aufweist.

Das erfindungsgemäße Kombinationspräparat wird vorzugsweise in eine kosmetische oder pharmazeutische Trägermatrix eingearbeitet, besonders bevorzugt in einer Einsatzkonzentration von 1,0 bis 5,0 Gew.-%. Bei der Trägermatrix kann es sich um Gelformulierungen, Cremeformulierungen (O/W- und W/O-Emulsionen), Lotionen, Maskenanwendungen etc. handeln.

Ein Verfahren zur Formulierung eines Kombinationspräparats gemäß der vorliegenden Erfindung in Form eines Gels ist in folgender Weise zu beschreiben: Unter leichtem Rühren werden ein Verdikker, vorzugsweise in einer Menge von 0,1 bis 3,0 Gew.-%, und ein nichtionischer Emulgator, vorzugsweise in einer Menge von 1,0 bis 15,0 Gew.-%, und bei einer besonders bevorzugten Ausführungsform ein Co-Emulgator in Wasser vollständig gelöst. In diese Matrix wird bei maximal 30°C eine der oben beschriebenen Ausführungsformen des erfindungsgemäßen Kombinationspräparats, vorzugsweise in einer Menge von 1,0 bis 5,0 Gew.-%, homogen eingerührt. Abschließend kann ein Konservierungsmittel, vorzugsweise in einer Menge von 0,1 bis 0,5 Gew.-%, zugesetzt und im weiteren homogen eingerührt werden. Das Gel zeigt klare bis trübe Erscheinungsform. Die Viskosität variiert in Abhängigkeit von Art und Einsatzkonzentration des verwendeten Verdickers. Der pH-Wert des Gels wird erforderlichenfalls mit herkömmlichen Mitteln auf etwa 5,5 bis 7,0 eingestellt.

Es wird darauf hingewiesen, daß sämtliche Merkmale, wie sie sich aus der vorliegenden Beschreibung, den Figuren und den Ansprüchen für einen Fachmann erschließen, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder derartige Kombinationen technisch unmöglich oder sinnlos wären. Dies gilt auch, wenn die einzelnen Merkmale oder Merkmalsgruppen konkret nur im Zusammenhang mit bestimmten weiteren Merkmalen beschrieben wurden. Auf die umfassende Darstellung sämtlicher denkbarer Merkmalskombinationen wird hier nur der Kürze und der Lesbarkeit der Beschreibung wegen verzichtet. Beispielhaft ergeben sich aber einige solcher Merkmalskombinationen sowie weitere Vorteile, Merkmale und Anwendungsmöglichkeiten der vorliegenden Erfindung aus den folgenden Beispielen und den dazugehörigen Figuren.

### Beispiel 1

Ein erfindungsgemäßes Kombinationspräparat wird nach dem oben genannten Verfahren hergestellt und umfaßt folgende Bestandteile:

| | | |
|---|---|---|
| 16,0 Gew.-% | Ethanol, unvergällt | |
| 10,0 Gew.-% | Phospholipide (Lecithin/PL80) | |
| 5,0 Gew.-% | L-Theanin (99%) | |
| 0,5 Gew.-% | Algenhydrolysat | |
| | | (15,8% Asparaginsäure |
| | | 14,7% Glutaminsäure |
| | | 10,5% Alanin |
| | | 10,5% Glycin |
| | | 7,4% Leucin |
| | | 7,3% Threonin |
| | | 6,9% Serin |
| | | 6,2% Prolin |
| | | 5,1% Phenylalanin) |
| 0,05 Gew.-% | Methylparaben | |
| 0,03 Gew.-% | Sorbinsäure | |
| 0,5 Gew.-% | Kaliumdihydrogenphosphat | |
| ad 100 Gew.-% | Wasser | |

Zunächst wurden die freie Aminosäure L-Theanin sowie die freien Aminosäuren des Algenhydrolysats bei 40°C vollständig in Wasser gelöst. Es entsteht eine klare hellbraune Lösung mit einem pH-Wert von 5.8 bis 6.2. Unter Turraxieren (= Homogenisierung bei 3.000 bis 10.000 U/min) wurde nun eine ethanolische Lecithinlösung, die bei etwa 50°C hergestellt worden war, langsam der wäßrigen Aminosäurelösung zugefügt und im Anschluß durch einen 200 nm Polycarbonatfilter extrudiert. Unter stetigem Homogenisieren wurde abschließend der Phosphatpuffer zugesetzt und solange weiter homogenisiert, bis eine leicht viskose, homogene Emulsion entstand. Die Vesikelgröße, ausgedrückt als Durchmesser der Liposomen-Hohlkugeln, wurde mit einem Zetamaster S der Firma Malvern Instruments, UK, nach dem Verfahren der Photonen-Korrelations-Spektroskopie (PCS) mit 152 nm bestimmt. Wurde der angestrebte pH-Wert nicht unmittelbar erreicht, so konnte er erforderlichenfalls mit NaOH-Lösung auf einen pH-Wert von 5,5 bis 7,0 eingestellt werden.

### Beispiel 2

Das erfindungsgemäße Kombinationspräparat nach Beispiel 1 wurde in einer Einsatzkonzentration von 5,0 Gew.-% in eine Gelformulierung eingearbeitet. Als Gelmatrix wurde hier Carbomergel verwendet. Die Einarbeitung erfolgte unter Rühren bei einer Temperatur von 30°C.

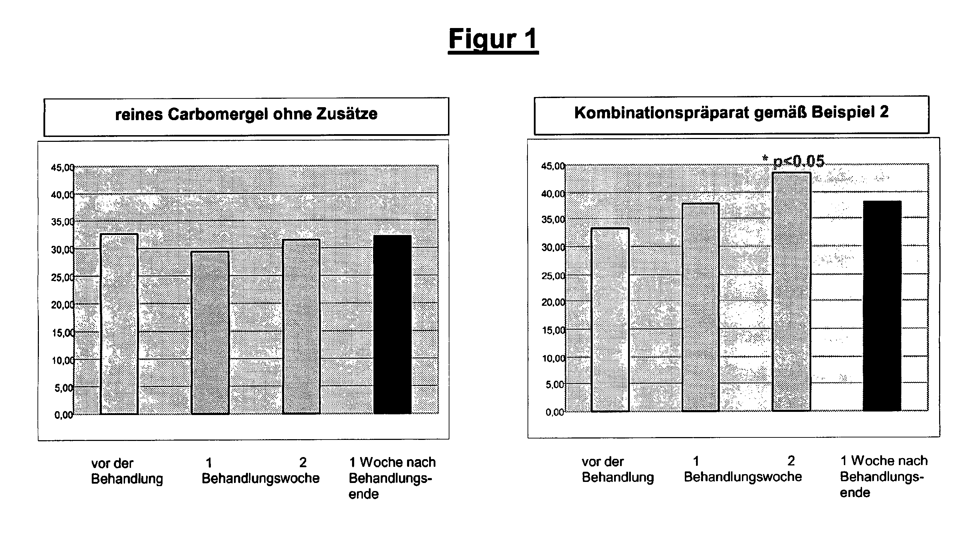

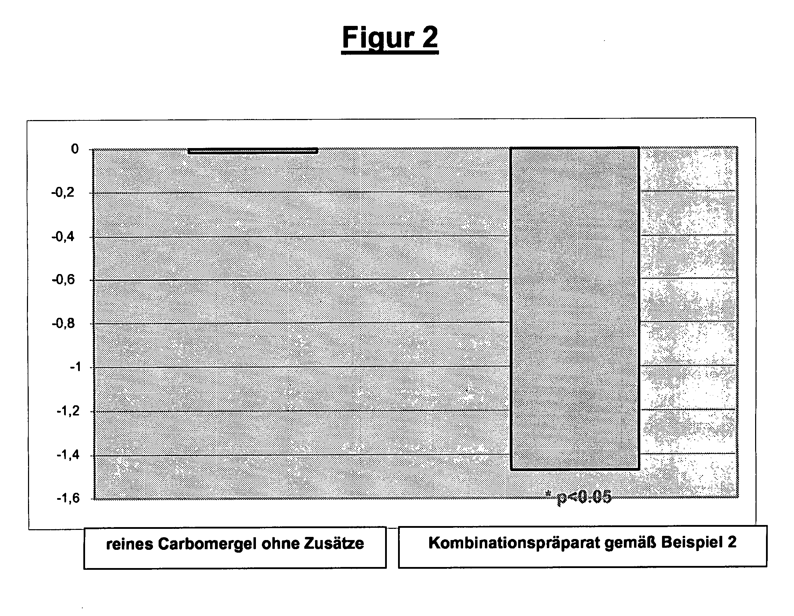

Das Ergebnis der Anwendung dieser bevorzugten Zubereitung des erfindungsgemäßen Kombinationspräparats ist in den folgenden Figuren 1 und 2 wiedergegeben.
- Figur 1: zeigt die Ergebnisse einer Untersuchung der feuchtigkeitsspendenden Eigenschaften eines Kombinationspräparats gemäß Beispiel 2.
- Figur 2: zeigt die Ergebnisse einer Untersuchung der elastizitätsverbessemden Eigenschaften eines erfindungsgemäßen Kombinationspräparats gemäß Beispiel 2.

Für die Studie, deren Ergebnisse in den Figuren 1 und 2 dargestellt sind, wurde ein erfindungsgemäßes Kombinationspräparat gemäß Beispiel 2 an sechs freiwilligen Versuchspersonen über einen Zeitraum von zwei Wochen getestet. Die Gelformulierung wurde zweimal täglich in Bereichen mit feinen Fältchen aufgetragen, nämlich um die Augen und auf der Innenseite des Unterarms. Vor der Behandlung, während des zweiwöchigen Zeitraums der Behandlung und eine Woche nach der Behandlung wurden folgende Parameter bestimmt: 1. die Hautfeuchtigkeit (Figur 1), 2. die Hautelastizität (Figur 2) und 3. die Anzahl und Tiefe der Falten (nicht dargestellt). Zur Bestimmung der Hautfeuchtigkeit wurden die behandelten Bereiche am inneren Unterarm mit Hilfe eines Corneometers bestimmt. In der Grafik auf der linken Seite von Figur 1 sind die gemessenen Werte (relative Größen) der Bereiche dargestellt, die mit reinem Carbomergel (ohne Wirkstoff) behandelt wurden. Auf der rechten Seite von Figur 1 sind dann die Ergebnisse der Hautbereiche dargestellt, die mit einem erfindungsgemäßen Kombinationspräparat gemäß Beispiel 2 behandelt wurden. Je höher die auf der Ordinate aufgetragenen Werte sind, desto höher ist auch die Hautfeuchtigkeit. Aus der linken Abbildung in Figur 1 geht hervor, daß die Behandlung mit reinem Carbomergel keine signifikante Veränderung, geschweige denn Verbesserung der Feuchtigkeit der mit dem Gel behandelten Hautpartien bewirkt.

Aus der Grafik auf der rechten Seite in Figur 1 ist ersichtlich, daß bereits nach einer Woche Behandlung mit erfindungsgemäßem Kombinationspräparat eine deutliche Verbesserung der Hautfeuchtigkeit festzustellen ist. Diese Verbesserung nimmt in der zweiten Woche noch einmal deutlich zu. Der Wert, der eine Woche nach Beendigung der Behandlung gemessen wurde, zeigt an, daß selbst zu diesem Zeitpunkt noch eine deutlich bessere Hautfeuchtigkeit vorlag als vor der Behandlung. Hieraus ist abzulesen, daß die Wirkung des erfindungsgemäßen Kombinationspräparats nicht nur kurzfristig, d.h. nur einen oder wenige Tage, anhält, sondern daß die Wirkung auch nach einer Woche noch andauert. Dieser Versuch belegt den großen Vorteil, den das erfindungsgemäße Kombinationspräparat den herkömmlichen Mitteln gegenüber hat, nämlich, daß es deutlich nachhaltiger wirkt. Das heißt, daß der Anwender, um den gewünschten Zustand zu erreichen, das Präparat nicht täglich anwenden muß, sondern daß auch eine Anwendung in regelmäßigen Abständen von beispielsweise drei oder vier Tagen oder auch einmal wöchentliche Anwendung eine deutliche Verbesserung gegenüber dem Zustand vor der Behandlung bringt.

In Figur 2 ist das Ergebnis des Elastizitätstests dargestellt, bei dem der Bereich mit feinen Fältchen in der Nähe der Augen mit einem Cutometer gemessen wurde. Die Messung nach zweiwöchiger Behandlung zeigt, daß sich der Spannungszustand der Haut durch Behandlung mit einem Kombinationspräparat gemäß der vorliegenden Erfindung deutlich von der Behandlung mit dem Matrixgel ohne den erfindungsgemäßen Zusatz von Aminosäuren abhebt. Je niedriger nämlich der Wert (relative Größe), der bei diesem Test erzielt wurde, desto besser ist die erzielte Elastizitätserhöhung der Haut.

Die Verminderung der Anzahl und der Tiefe der feinen Fältchen durch zweiwöchige Behandlung mit dem erfindungsgemäßen Kombinationspräparat wurde optisch ausgewertet. Hierbei konnte eine objektive Verbesserung gegenüber der Behandlung mit der reinen Gelmatrix festgestellt werden. Das heißt, daß die Behandlung mit dem erfindungsgemäßen Kombinationspräparat zu einer sichtbaren Verringerung der Anzahl der Falten geführt hat und gleichzeitig auch eine Verminderung der Faltentiefe (hautglättende Wirkung) der noch feststellbaren Fältchen bewirkt hat.

## Patentansprüche

1. Kosmetisches oder therapeutisches Kombinationspräparat mit einem Anteil an im wesentlichen freien Aminosäuren, wobei der Anteil an im wesentlichen freien Aminosäuren wenigstens Theanin, Asparaginsäure, Glutaminsäure, Glycin, Threonin, Alanin und Serin umfaßt, wobei der Anteil an Theanin bezogen auf den Gesamtanteil der im wesentlichen freien Aminosäuren im Präparat 80 bis 95 Gew.-% beträgt und wobei der Anteil an Asparaginsäure, Glutaminsäure, Glycin, Threonin, Alanin und Serin bezogen auf den Gesamtanteil der im wesentlichen freien Aminosäuren Im Präparat insgesamt 5 bis 20 Gew.-% beträgt.

2. Kombinationspräparat nach Anspruch 1, **dadurch gekennzeichnet, daß** es ein Algenhydrolysat oder das Hydrolysat eines Algenextrakts umfaßt, vorzugsweise ein Grünalgenhydrolysat oder das Hydrolysat eines Grünalgenextrakts umfaßt.

3. Kombinationspräparat nach einem der Ansprüche 1 bis 2, **dadurch gekennzeichnet, daß** es ein Hydrolysat einer Grünalge oder eines Extrakts einer Grünalge der Gattung Enteromorpha, vorzugsweise ein Hydrolysat der Grünalge oder des Extrakts der Grünalge *Enteromorpha Intestinalis* umfaßt.

4. Kombinationspräparat nach einem der Ansprüche 2 oder 3, **dadurch gekennzeichnet, daß** das Algenhydrolysat oder das Hydrolysat des Algenextrakts im wesentlichen frei von Salzen Ist.

5. Kombinationspräparat nach einem der Ansprüche 1 bis 4, **dadurch gekennzeichnet, daß** der Anteil an im wesentlichen freien Aminosäuren bezogen auf das fertige Präparat 0,5 bis 10 Ges.-%, vorzugsweise 3 bis 6 Gew.-%, besonders bevorzugt etwa 5 Gew.-% beträgt.

6. Kombinationspräparat nach einem der Ansprüche 1 bis 5, **dadurch gekennzeichnet, daß** der Anteil an Theanin bezogen auf den Gesamtanteil der im wesentlichen freien Aminosäuren im Präparat 85 bis 95 Gew.-%, besonders bevorzugt 90 bis 94 Gew.-% beträgt.

7. Kombinationspräparat nach einem der Ansprüche 1 bis 6, **dadurch gekennzeichnet, daß** der Anteil der im wesentlichen freien Aminosäuren, die in Anspruch 1 aufgezählt sind, und die von Theanin verschieden sind, bezogen auf den Gesamtanteil der im wesentlichen freien Aminosäuren im Präparat 5 bis 15 Gew.-%, besonders bevorzugt 6 bis 10 Gew.-% beträgt.

8. Kombinationspräparat nach einem der Ansprüche 1 bis 7, **dadurch gekennzeichnet, daß** der Anteil der im wesentlichen freien Aminosäuren, die von Theanin verschieden sind,
a) 13 bis 18 Gew.-% Asparaginsäure,
b) 12 bis 17 Gew.-% Glutaminsäure und
c) 8 bis 12 Gew.-% Alanin
enthält.

9. Kombinationspräparat nach einem der Ansprüche 1 bis 8, **dadurch gekennzeichnet, daß** das Präparat einen kosmetisch verträglichen Träger umfaßt.

10. Kombinationspräparat nach Anspruch 9, **dadurch gekennzeichnet, daß** der Träger membranbildende Lipide umfaßt, wobei vorzugsweise die membranbildenden Lipide vesikulär vorliegen.

11. Kombinationspräparat nach einem der Ansprüche 9 und 10, **dadurch gekennzeichnet, daß** die membranbildenden Lipide unter Phospholipiden, Ceramiden und Diacylglykosiden und Gemischen davon ausgewählt sind.

12. Kombinationspräparat nach einem der Ansprüche 9 bis 11, **dadurch gekennzeichnet, daß** die membranbildenden Lipide wenigstens 70 Gew.-% Phosphatidylcholin, vorzugsweise etwa 80 bis 90 Gew.-% Phosphatidylcholin enthalten.

13. Kombinationspräparat nach einem der Ansprüche 9 bis 12, **dadurch gekennzeichnet, daß** der Träger weiterhin Linolsäure in stabilisierter Form, vorzugsweise in einer Menge von 3,5 bis 6,5 Gew.-% enthält.

14. Kombinationspräparat nach einem der Ansprüche 1 bis 13, **dadurch gekennzeichnet, daß** es wenigstens einen kosmetisch verträglichen Hilfsstoff umfaßt, der unter Methylparaben, Sorbinsäure und Kaliumdihydrogenphosphat ausgewählt ist.

15. Kombinationspräparat nach einem der Ansprüche 1 bis 14, **dadurch gekennzeichnet, daß** die im wesentlichen freien Aminosäuren im Präparat an einen Trägerstoff oder mehrere Trägerstoffe gebunden vorliegen, wobei der Trägerstoff oder die Trägerstoffe so ausgewählt ist/sind, daß er/sie die Aminosäuren nach der Applikation des Präparates wieder freisetzt.

16. Verwendung von im wesentlichen freien Aminosäuren zur Herstellung eines Kosmetikums für die Verbesserung der Hautfeuchtigkeit, der Hautelastizität und/oder für die Hautfaltenreduzierung, wobei das Kosmetikum einen Anteil an im wesentlichen freien Aminosäuren aufweist, wobei der Anteil an im wesentlichen freien Aminosäuren wenigstens Theanin, Asparaginsäure, Glutaminsäure, Glycin, Threonin, Alanin und Serin umfaßt, wobei der Anteil an Theanin bezogen auf den Gesamtanteil der im wesentlichen freien Aminosäuren im Präparat 80 bis 95 Gew.-% beträgt und wobei der Anteil an Asparaginsäure, Glutaminsäure, Glycin, Threonin, Alanin und Serin bezogen auf den Gesamtanteil der im wesentlichen freien Aminosäuren im Präparat insgesamt 5 bis 20 Gew.-% beträgt.

17. Verfahren zur Herstellung eines kosmetischen oder therapeutischen Kombinationspräparats nach einem der Ansprüche 2 bis 15, bei dem man die Aminosäuren durch enzymatische Spaltung von Algenproteinen gewinnt, vorzugsweise durch die enzymatische Spaltung von Grünalgenproteinen und besonders bevorzugt durch die enzymatische Spaltung von Grünalgen der Gattung *Enteromorpha.*

## Claims

1. A cosmetic or therapeutic combination preparation comprising a proportion of substantially free amino acids, wherein the proportion of substantially free amino acids contains at least theanine, aspartic acid, glutamic acid, glycine, threonine, alanine and serine, wherein the proportion of theanine with respect to the total proportion of the substantially free amino acids in the preparation is 80 to 95% by weight, and wherein the proportion of aspartic acid, glutamic acid, glycine, threonine, alanine and serine with respect to the total proportion of the substantially free amino acids in the preparation is 5 to 20% by weight.

2. A combination preparation according to claim 1 **characterised in that** it includes an alga hydrolysate or the hydrolysate of an alga extract, preferably a green alga hydrolysate or the hydrolysate of a green alga extract.

3. A combination preparation according to one of claims 1 to 2 **characterised in that** it includes a hydrolysate of a green alga or an extract of a green alga of the genus Enteromorpha, preferably a hydrolysate of the green alga or the extract of the green alga *Enteromorpha intestinalis.*

4. A combination preparation according to one of claims 2 and 3 **characterised in that** the alga hydrolysate or the hydrolysate of the alga extract is substantially free from salts.

5. A combination preparation according to one of claims 1 to 4 **characterised in that** the proportion of substantially free amino acids with respect to the finished preparation is 0.5 to 10% by weight, preferably 3 to 6% by weight, particularly preferably about 5% by weight.

6. A combination preparation according to one of claims 1 to 5, **characterised in that** the proportion of theanine with respect to the total proportion of the substantially free amino acids in the preparation is 85 to 95% by weight, particularly preferably 90 to 94% by weight.

7. A combination preparation according to one of claims 1 to 6 **characterised in that** the proportion of the substantially free amino acids which are recited in claim 1 and which are different from theanine, with respect to the total proportion of the substantially free amino acids in the preparation, is 5 to 15% by weight, particularly preferably 6 to 10% by weight.

8. A combination preparation according to one of claims 1 to 7 **characterised in that** the proportion of the substantially free amino acids which are different from theanine includes
a) 13 to 18% by weight of aspartic acid,
b) 12 to 17% by weight of glutamic acid, and
c) 8 to 12% by weight of alanine.

9. A combination preparation according to one of claims 1 to 8 **characterised in that** the preparation includes a cosmetically compatible carrier.

10. A combination preparation according to claim 9 **characterised in that** the carrier includes membrane-forming lipids, wherein preferably the membrane-forming lipids are present in vesicular form.

11. A combination preparation according to one of claims 9 and 10 **characterised in that** the membrane-forming lipids are selected from phospholipids, ceramides and diacylglycosides and mixtures thereof.

12. A combination preparation according to one of claims 9 to 11 **characterised in that** the membrane-forming lipids contain at least 70% by weight of phosphatidyl choline, preferably about 80 to 90% by weight of phosphatidyl choline.

13. A combination preparation according to one of claims 9 to 12 **characterised in that** the carrier further includes linoleic acid in stabilised form, preferably in an amount of 3.5 to 6.5% by weight.

14. A combination preparation according to one of claims 1 to 13 **characterised in that** it includes at least one cosmetically compatible additive which is selected from methylparaben, sorbic acid and potassium dihydrogen phosphate.

15. A combination preparation according to one of claims 1 to 14 **characterised in that** the substantially free amino acids in the preparation are present bonded to a carrier substance or a plurality of carrier substances, wherein the carrier substance or substances is/are so selected that it/they again liberates/liberate the amino acids after the application of the preparation.

16. Use of substantially free amino acids for the production of a cosmetic for improving skin moistness, skin elasticity and/or for skin wrinkle reduction, wherein the cosmetic contains a proportion of substantially free amino acids, wherein the proportion of substantially free amino acids contains at least theanine, aspartic acid, glutamic acid, glycine, threonine, alanine and serine, wherein the proportion of theanine with respect to the total proportion of the substantially free amino acids in the preparation is 80 to 95% by weight, and wherein the proportion of aspartic acid, glutamic acid, glycine, threonine, alanine and serine with respect to the total proportion of the substantially free amino acids in the preparation is 5 to 20% by weight.

17. A method of producing a cosmetic or therapeutic combination preparation according to one of claims 2 to 15 in which the amino acids are obtained by enzymatic cleaving of alga proteins, preferably by enzymatic cleaving of green alga proteins and particularly preferably by enzymatic cleaving of green algae of the genus *Enteromorpha.*

## Revendications

1. Préparation combinée cosmétique ou thérapeutique présentant un pourcentage d'acides aminés sensiblement libres, le pourcentage d'acides aminés sensiblement libres comprenant au moins de la théanine, de l'acide asparaginique, de l'acide glutamique, de la glycine, de la thréonine, de l'alanine et de la sérine, le pourcentage de théanine rapporté au pourcentage total d'acides aminés sensiblement libres dans la préparation étant de 80 à 95% en poids, et le pourcentage d'acide asparaginique, d'acide glutamique, de glycine, de thréonine, d'alanine et de sérine rapporté au pourcentage total d'acides aminés sensiblement libres dans la préparation étant au total de 5 à 20% en poids.

2. Préparation combinée selon la revendication 1, **caractérisée en ce qu'**elle comprend un hydrolysat d'algue ou l'hydrolysat d'un extrait d'algue, avantageusement un hydrolysat d'algue verte ou l'hydrolysat d'un extrait d'algue verte.

3. Préparation combinée selon l'une des revendications 1 à 2, **caractérisée en ce qu'**elle comprend un hydrolysat d'une algue verte ou un extrait d'une algue verte du genre Enteromorpha, avantageusement un hydrolysat de l'algue verte ou de l'extrait de l'algue verte Enteromorpha Intestinalis.

4. Préparation combinée selon l'une des revendications 2 ou 3, **caractérisée en ce que** l'hydrolysat d'algue ou l'hydrolysat de l'extrait d'algue est sensiblement dépourvu de sels.

5. Préparation combinée selon l'une des revendications 1 à 4, **caractérisée en ce que** le pourcentage d'acides aminés sensiblement libres rapporté à la préparation finale est de 0,5 à 10% en poids, avantageusement de 3 à 6% en poids, de façon particulièrement préférée environ 5% en poids.

6. Préparation combinée selon l'une des revendications 1 à 5, **caractérisée en ce que** le pourcentage de théanine rapporté au pourcentage total d'acides aminés sensiblement libres dans la préparation est de 85 à 95% en poids, de façon particulièrement préférée de 90 à 94% en poids.

7. Préparation combinée selon l'une des revendications 1 à 6, **caractérisée en ce que** le pourcentage d'acides aminés sensiblement libres, mentionnés à la revendication 1 et autres que la théanine, rapporté au pourcentage total d'acides aminés sensiblement libres dans la préparation est de 5 à 15% en poids, de façon particulièrement préférée de 6 à 10% en poids.

8. Préparation combinée selon l'une des revendications 1 à 7, **caractérisée en ce que** le pourcentage d'acides aminés sensiblement libres, autres que la théanine, contient
a) 13 à 18% en poids d'acide asparaginique,
b) 12 à 17% en poids d'acide glutamique et
c) 8 à 12% en poids d'alanine.

9. Préparation combinée selon l'une des revendications 1 à 8, **caractérisée en ce que** la préparation comprend un support acceptable du point de vue cosmétique.

10. Préparation combinée selon la revendication 9, **caractérisée en ce que** le support comprend des lipides membranaires, les lipides membranaires se présentant avantageusement sous forme vésiculaire.

11. Préparation combinée selon l'une des revendications 9 et 10, **caractérisée en ce que** les lipides membranaires sont choisies parmi les phospholipides, les céramides et les diacylglycosides et des mélanges de ceux-ci.

12. Préparation combinée selon l'une des revendications 9 à 11, **caractérisée en ce que** les lipides membranaires contiennent au moins 70% en poids de phosphatidylcholine, avantageusement environ 80 à 90% en poids de phosphatidylcholine.

13. Préparation combinée selon l'une des revendications 9 à 12, **caractérisée en ce que** le support contient en outre des acides linoléiques sous forme stabilisée, avantageusement dans une quantité de 3,5 à 6,5% en poids.

14. Préparation combinée selon l'une des revendications 1 à 13, **caractérisée en ce qu'**elle comprend au moins une substance auxiliaire acceptable du point de vue cosmétique qui est choisie parmi le méthylparaben, l'acide sorbique et le dihydrogénophosphate de potassium.

15. Préparation combinée selon l'une des revendications 1 à 14, **caractérisée en ce que** les acides aminés sensiblement libres dans la préparation se présentent sous une forme liée à un support ou plusieurs supports, le support ou les supports étant choisis de façon à libérer de nouveau les acides aminés après l'application de la préparation.

16. Utilisation d'acides aminés sensiblement libres dans la fabrication d'un cosmétique destiné à améliorer l'humidité de la peau, l'élasticité de la peau et/ou à réduire les rides de la peau, le cosmétique présentant un pourcentage d'acides aminés sensiblement libres, le pourcentage d'acides aminés sensiblement libres comprenant au moins de la théanine, de l'acide asparaginique, de l'acide glutamique, de la glycine, de la thréonine, de l'alanine et de la sérine, le pourcentage de théanine rapporté au pourcentage total d'acides aminés sensiblement libres dans la préparation étant de 80 à 95% en poids, et le pourcentage d'acide asparaginique, d'acide glutamique, de glycine, de thréonine, d'alanine et de sérine rapporté au pourcentage total d'acides aminés sensiblement libres dans la préparation étant au total de 5 à 20% en poids.

17. Procédé de fabrication d'une préparation combinée cosmétique ou thérapeutique selon l'une des revendications 2 à 15, dans lequel on obtient les acides aminés par clivage enzymatique de protéines d'algue, avantageusement par clivage enzymatique de protéines d'algue verte et de façon particulièrement préférée par clivage enzymatique d'algue verte du genre Enteromorpha.
